# EUROPÄISCHE PATENTSCHRIFT

(11) **EP 1 478 284 B1**
(45) Veröffentlichungstag und Bekanntmachung des Hinweises auf die Patenterteilung: **31.08.2005**
(21) Anmeldenummer: 03742509.7
(22) Anmeldetag: 06.02.2003
(51) Int. Cl.: A61B 17/17

(54) **FÜHRUNG FÜR EIN DREHBARES CHIRURGISCHES BEARBEITUNGSWERKZEUG**
GUIDE FOR A ROTATING SURGICAL MACHINING TOOL
GUIDE POUR OUTIL D'USINAGE CHIRURGICAL ROTATIF

(30) Priorität: 20.02.2002 DE 10207034
(43) Veröffentlichungstag der Anmeldung: 24.11.2004
(73) Patentinhaber: Aesculap AG & Co. KG, 78532 Tuttlingen (DE)
(72) Erfinder: BÖTTIGER, Roland, D-78604 Rietheim-Weilheim (DE); HAGEN, Thomas, D-78532 Tuttlingen (DE)
(74) Vertreter: Boehme, Ulrich
(86) Internationale Anmeldenummer: PCT/EP2003/001152
(87) Internationale Veröffentlichungsnummer: WO 2003/070111

(56) Entgegenhaltungen:
- EP-A- 0 570 076
- FR-A- 2 721 202
- US-A- 5 569 256
- US-A- 5 653 714

## Beschreibung

Die Erfindung betrifft eine Führung für ein mittels einer Bearbeitungsschablone in einer Schlitzführung geführtes drehbares chirurgisches Bearbeitungswerkzeug.

Bei chirurgischen Operationen müssen häufig Knochen bearbeitet werden, beispielsweise beim Ersetzen einer Gelenkfläche durch ein künstliches Gelenkimplantat. Es ist bekannt, dazu oszillierende Sägen zu verwenden, die in Führungsschlitzen von Bearbeitungsschablonen geführt werden, die in geeigneter Weise am Knochen festgelegt sind.

Es ist auch bekannt, die Bearbeitung mit rotierenden Fräsern vorzunehmen, die in der Regel relativ geringe Drehzahlen aufweisen und die dann über kreiszylindrische Umfangsflächen direkt an Führungsschlitzen von Bearbeitungsschablonen geführt werden können (EP 1 084 679 A2). Eine solche Führung hat jedoch den Nachteil, daß die Drehflächen des Fräsers direkt an den Führungsflächen des Führungsschlitzes anliegen, und dabei kommt es zu Abnutzungserscheinungen und gegebenenfalls zu unzulässig hoher Erwärmung im Berührungsbereich. Dies läßt sich bei langsam drehenden Fräsern tolerieren, ist aber bei schnelldrehenden Bearbeitungswerkzeugen nicht mehr möglich.

Eine weitere derartige Führung ist in der EP 0 570 076 A1 offenbart.

Es ist Aufgabe der Erfindung, eine gattungsgemäße Führung so auszugestalten, daß auch mit schnell drehenden rotierenden Fräsern gearbeitet werden kann, ohne daß unzulässige Erwärmungen und Abnutzungserscheinungen auftreten.

Diese Aufgabe wird bei einer Führung der eingangs beschriebenen Art erfindungsgemäß dadurch gelöst, daß sie einen Führungskörper umfaßt, dessen Höhe der der Schlitzführung entspricht, während seine Breite geringer ist als die Breite der Schlitzführung, daß der Führungskörper eine durchgehende Aufnahmebohrung für das Bearbeitungswerkzeug aufweist und daß in der Aufnahmebohrung mindestens eine an dem Bohrwerkzeug anliegende Führungsfläche angeordnet ist, die koaxial zur Drehachse des Bohrwerkzeuges drehbar in dem Führungskörper gelagert ist.

Bei dieser Ausgestaltung wird also das Bohrwerkzeug nicht unmittelbar an den einander gegenüberliegenden Flächen der Schlitzführung angelegt, sondern in die Schlitzführung wird ein Führungskörper eingesetzt, der zwischen den einander gegenüberliegenden Flächen der Schlitzführung geführt wird, in der Schlitzführung dagegen quer zu sich selbst verschiebbar ist. In diesem Führungskörper wird das Bohrwerkzeug geführt, durch eine Führungsfläche, die relativ zu dem Führungskörper drehbar ist, und zwar koaxial zur Drehachse des Bohrwerkzeuges. Damit kann sich diese Führungsfläche mit dem Bohrwerkzeug mitdrehen, so daß die relative Bewegung der Umfangsfläche des Bohrwerkzeuges gegenüber der Führungsfläche herabgesetzt wird, gegebenenfalls kann diese Relativbewegung ganz verschwinden.

Durch Verwendung eines derartigen Führungskörpers wird es möglich, auch mit sehr schnell drehenden Fräswerkzeugen längere Zeit und ohne unzulässige Erwärmung zu arbeiten, beispielsweise mit Hochgeschwindigkeits-Fingerfräsern.

Günstig ist es, wenn in der Aufnahmebohrung zwei drehbare Führungsflächen im Abstand zueinander angeordnet sind, dadurch wird eine sichere Führung des Bohrwerkzeuges im Führungskörper erreicht.

Bei einer besonders bevorzugten Ausführungsform sind drei derartige Führungsflächen im Abstand angeordnet, dadurch wird sichergestellt, daß ein Bearbeitungswerkzeug auch dann zuverlässig in dem Führungskörper geführt wird, wenn es unterschiedlich tief in die Aufnahmebohrung eintaucht, so daß möglicherweise Schneidflächen im Bereich der Führungsflächen angeordnet sind, deren Umfangskontur von der maximalen Umfangskontur des Bohrwerkzeuges abweichen kann. Durch die Verwendung von drei Führungsflächen ist sichergestellt, daß immer mindestens zwei Bereiche mit maximaler Umfangsfläche an einer der drehbaren Führungsflächen anliegt.

Besonders vorteilhaft ist es, wenn die Führungsfläche durch die Innenwand eines inneren Lagerringes eines Wälzlagers gebildet wird, insbesondere eines Kugellagers.

Bei einer bevorzugten Ausführungsform ist vorgesehen, daß der Führungskörper an seiner Oberseite und/oder an seiner Unterseite Reibflächen aufweist, die reibend an den einander gegenüberliegenden Flächen der Schlitzführung der Bearbeitungsschablone anliegen. Diese Reibflächen verhindern, daß der Führungskörper unbeabsichtigt in axialer Richtung aus dem Führungsschlitz herausfällt, sie fixieren also den Führungskörper im Innern der Schlitzführung, ohne jedoch eine Verschiebung des Führungskörpers längs des Schlitzes und/oder eine Verschwenkung in der Ebene des Schlitzes zu verhindern.

Insbesondere können die Reibflächen durch elastisch zusammendrückbare Teilbereiche der Oberseite und/oder der Unterseite des Führungskörpers gebildet werden, insbesondere durch gummielastische O-Ringe, die in quer zur Längsrichtung des Führungskörpers umlaufenden Umfangsnuten des Führungskörpers eingelegt sind.

Bei einer bevorzugten Ausführungsform kann vorgesehen werden, daß der Führungskörper durch mindestens einen in einen Rücksprung eingreifenden Vorsprung relativ zum Führungsschlitz gegen eine Verschiebung in axialer Richtung gesichert ist. Trotzdem bleibt der Führungskörper in der Ebene der Schlitzführung quer verschiebbar.

Insbesondere kann vorgesehen sein, daß der Rücksprung und der Vorsprung derart ineinander eingreifen, daß der Führungskörper relativ zum Führungsschlitz quer zur Längsrichtung verschiebbar und um eine senkrecht auf dem Führungsschlitz stehende Drehachse verschwenkbar ist. Ein in den Führungskörper eingesetztes Bearbeitungswerkzeug kann also vom Operateur in alle Stellungen einer Bearbeitungsebene gebracht werden, sei es durch Querverschiebung des Führungskörpers, sei es durch Verschwenkung desselben, sei es durch Überlagerung beider Bewegungen.

Beispielsweise kann der Vorsprung eine flanschförmige Verbreiterung am Führungskörper sein, die in eine parallel zum Führungsschlitz verlaufende Nut an der Bearbeitungsschablone eintaucht.

Dabei ist es vorteilhaft, wenn die Verbreiterung derart abgeschrägt ist, daß der Führungskörper in der Ebene des Führungsschlitzes relativ zu der Nut verschwenkbar ist. Durch die Abschrägung verkantet sich die flanschförmige Verbreiterung nicht in der Nut, wenn der Führungskörper gegenüber der Nut verschwenkt wird, dabei reichen in der Praxis Schwenkwinkel in der Größenordnung bis zu ±30° aus.

Bei einer solchen Ausgestaltung ist es weiterhin von Vorteil, wenn die Seitenwand der Nut an einer Stelle fehlt, so daß die Verbreiterung an dieser Stelle seitlich aus der Nut austreten kann. Damit erhält man die Möglichkeit, den Führungskörper an dieser Stelle aus dem Führungsschlitz in axialer Richtung herauszuziehen, an jeder anderen Stelle der Nut wird jedoch dieses Herausziehen durch den Eingriff der Verbreiterung in die Nut verhindert.

Bei einer weiteren bevorzugten Ausführungsform ist vorgesehen, daß der Führungskörper an seinen beiden Enden aus dem Führungsschlitz hervorragt und dort Verbreiterungen trägt, die den Führungskörper gegen eine axiale Verschiebung im Führungsschlitz sichern. Dabei ist es vorteilhaft, wenn die Verbreiterungen mit Spiel an der Vorder- und Rückseite des Führungsschlitzes angeordnet sind, so daß der Führungskörper in der Ebene des Führungsschlitzes um eine senkrecht auf dieser Ebene stehende Drehachse verschwenkbar ist, auch hier reicht eine Verschwenkung in der Größenordnung von ±30°.

Um den Führungskörper auch bei einer solchen Ausgestaltung aus dem Führungsschlitz entnehmen zu können, kann vorgesehen sein, daß der Führungsschlitz an einer Stelle einen vergrößerten Abstand der einander gegenüberliegenden Flächen aufweist, so daß an dieser Stelle zumindest eine der Verbreiterungen durch den Führungsschlitz hindurchtreten kann.

Vorzugsweise ist der Führungskörper als kreiszylindrische Führungshülse ausgebildet.

Es kann dabei vorgesehen sein, daß der Führungskörper einen von der kreisförmigen Querschnittsform abweichenden Sicherungsabschnitt aufweist, der an einer Anlagefläche der Bearbeitungsschablone anliegt und eine Verdrehung des Führungskörpers um seine Längsachse verhindert.

Der Sicherungsabschnitt kann beispielsweise an der flanschförmigen Verbreiterung angeordnet sein, die in die Nut eintaucht, und an der Nut anliegen.

Bei einer anderen Ausführungsform ist vorgesehen, daß der Führungskörper an der Oberseite und/oder der Unterseite abgeflacht ausgebildet ist und daß diese an den gegenüberliegenden Flächen des Führungsschlitzes anliegenden abgeflachten Bereiche den Sicherungsabschnitt bilden.

Die nachfolgende Beschreibung bevorzugter Ausführungsformen der Erindung dient im Zusammenhang mit der Zeichnung der näheren Erläuterung. Es zeigen:
- Figur 1:: eine Seitenansicht einer an einem Knochen festgelegten Bearbeitungsschablone mit einem ebenen Führungsschlitz und einem darin aufgenommenen Führungskörper mit eingeführtem Fräswerkzeug;
- Figur 2:: eine perspektivische Seitenansicht eines Teils der in Figur 1 dargestellten Bearbeitungsschablone mit zwei in gegeneinander geneigten Ebenen angeordneten Führungsschlitzen und mit einem in einen Führungsschlitz eingesetzten Führungskörper vor dem Einführen des Bearbeitungsfräsers in den Führungskörper;
- Figur 3:: eine Schnittansicht längs Linie 3-3 in Figur 2;
- Figur 4:: eine Schnittansicht ähnlich Figur 3 bei einem abgewandelten Ausführungsbeispiel eines Führungskörpers mit Verbreiterungen an beiden aus dem Führungsschlitz hervorragenden Enden und
- Figur 5:: eine Draufsicht auf ein besonderes Ausführungsbeispiel eines Führungsschlitzes mit einer verbreiterten Einschubstelle für den Führungskörper.

Die Erfindung wird am Beispiel einer Bearbeitungsschablone erläutert, die an einem Röhrenknochen befestigt ist, zum Beispiel am Femur, und mit deren Hilfe der Gelenkkopf des Femurs bearbeitet werden soll. Es versteht sich aber, daß die Erfindung auch bei Bearbeitungsschablonen Verwendung finden kann, die an anderen Stellen des Körpers zur Bearbeitung eines Knochens eingesetzt werden.

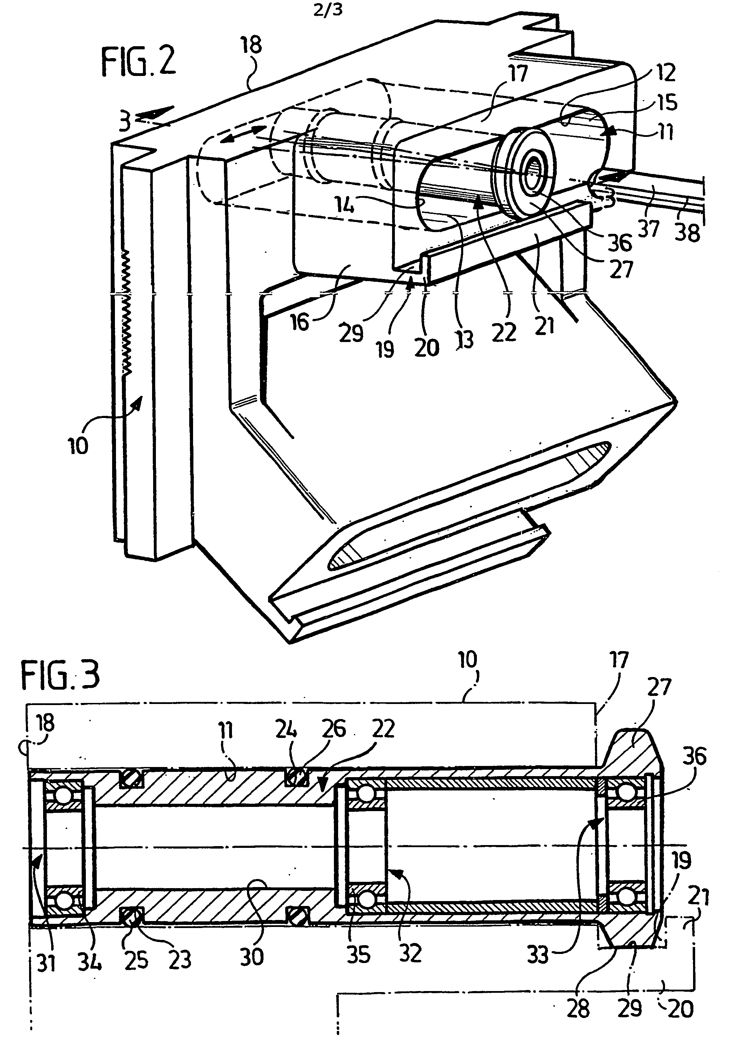

Bei dem in der Zeichnung dargestellten Ausführungsbeispiel ist an einem Knochen 1 eine Bearbeitungsschablone 2 festgelegt, und zwar mit Hilfe von in den Knochen eingeschraubten Knochenschrauben 3. Bei dem dargestellten Ausführungsbeispiel sind an den Knochenschrauben 3 über Klemmeinrichtungen 4 Haltestäbe 5 befestigt, die ihrerseits eine Plattform 6 tragen. An dieser ist über mehrere Verstelleinrichtungen 7, 8, 9 relativ zur Plattform 6 verstellbar ein Führungsblock 10 gehalten, der in Figur 2 vergrößert dargestellt ist. Dieser Führungsblock 10 weist an seinem oberen Ende einen länglichen Führungsschlitz 11 auf mit zwei parallelen, ebenen, einander gegenüberliegenden Seitenflächen 12, 13, die über kreisbogenförmig gebogene Endflächen 14, 15 ineinander übergehen. Der Führungsschlitz 11 durchsetzt einen schachtförmigen Vorsprung 16 des Führungsblockes 10 und erstreckt sich von seiner Vorderseite 17 zu seiner Rückseite 18 über eine größere Länge, beispielsweise ist der Abstand von Vorderseite zu Rückseite so groß wie die Breite des Führungsschlitzes.

An der Vorderseite 17 ist unterhalb des Führungsschlitzes 11 eine an dessen unterer Seitenfläche 13 entlanglaufende, zum Führungsschlitz 11 hin offene Nut 19 in einem von der Vorderseite 17 abstehenden Vorsprung 20 angeordnet, diese erstreckt sich im wesentlichen über die gesamte Breite des Führungsschlitzes 11, allerdings fehlt an einem Endbereich der Nut 19 ein kurzer Teil der Seitenwand 21 der Nut, wie dies aus der Darstellung der Figur 2 deutlich wird.

Der Führungsblock 10 weist in dem dargestellten Ausführungsbeispiel einen zweiten, vollständig gleich ausgebildeten Führungsschlitz auf, dessen Ebene gegenüber der Ebene des ersten Führungsschlitzes 11 geneigt ist, da dieser vollständig gleich aufgebaut ist und auch in gleicher Weise verwendet wird, beschränkt sich die Beschreibung auf den oberen Führungsschlitz 11.

In den Führungsschlitz 11 ist ein Führungskörper 22 in Form einer kreiszylindrischen Hülse eingeschoben, deren Außendurchmesser dem Abstand der beiden Seitenflächen 12 und 13 des Führungsschlitzes 11 entspricht. In zwei Umfangsnuten 23, 24 des Führungskörpers 22 sind O-Ringe 25, 26 aus gummielastischem Material eingelegt, so daß der Führungskörper 22 im Reibsitz im Führungsschlitz 11 gehalten ist. An seinem der Vorderseite 17 benachbarten Ende trägt der Führungskörper 22 eine ringflanschförmige Verbreiterung 27, die in die Nut 19 eintaucht und die dadurch den Führungskörper 22 in axialer Richtung gegen eine Verschiebung im Führungsschlitz 11 sichert. Die Verbreiterung 27 ist dabei seitlich abgeflacht, so daß der Führungskörper 22 nicht nur quer zu seiner Längsrichtung im Führungsschlitz 11 frei verschiebbar ist, sondern auch in der Ebene des Führungsschlitzes 11 um eine senkrecht zu dieser Ebene verlaufende Drehachse verschwenkbar ist, der Verschwenkwinkel liegt dabei in der Größenordnung bis zu ±30°.

An einer Seite weist die Verbreiterung 27 eine Abflachung 28 auf, d. h. in diesem Bereich weicht der Querschnitt der ringflanschförmigen Verbreiterung 27 von der Kreisform ab. Diese Abflachung 28 taucht in die Nut 19 ein und liegt dort am Boden 29 der Nut 19 derart an, daß eine Verdrehung des Führungskörpers 22 um seine Längsachse verhindert wird.

Diese Anordnung ermöglicht es, den Führungskörper verdrehungsfrei längs der Ebene des Führungsschlitzes 11 zu verschieben und zu verschwenken, ohne daß der Führungskörper 6 in axialer Richtung aus dem Führungsschlitz 11 herausgezogen werden kann. Lediglich in dem seitlichen Endbereich der Nut 19, in dem die Seitenwand 21 fehlt, kann die ringflanschförmige Verbreiterung 27 seitlich aus der Nut 19 austreten, d. h. in diesem Bereich kann man den Führungskörper 22 in axialer Richtung in den Führungsschlitz 11 hineinschieben bzw. aus diesem wieder herausziehen. Damit er nicht unbeabsichtigt aus dem Führungsschlitz 11 herausgleitet, wenn die ringflanschförmige Verbreiterung 27 in diesem Teilbereich der Nut 19 ohne Seitenwand 21 angeordnet ist, sind die O-Ringe 25 und 26 vorgesehen, die den Führungskörper 22 im Reibsitz in dem Führungsschlitz 11 festhalten, die aber eine normale seitliche Verschiebung und Verschwenkung des Führungskörpers 22 im Führungsschlitz 11 nicht ernsthaft behindern.

Im Inneren des hülsenförmigen Führungskörpers 22 ist eine durchgehende Bohrung 30 vorgesehen, in der im Abstand zueinander drei Kugellagerringe 31, 32, 33 angeordnet sind, und zwar jeweils ein derartiger Kugellagerring an beiden Enden des Führungskörpers 22 und einer in der Mitte. Die Kugellagerringe 31, 32, 33 sind durch geeignete, hier nicht näher erläuterte Mittel in axialer Richtung festgelegt, die inneren Lagerringe 34, 35, 36 weisen alle denselben Innendurchmesser auf, alle Kugellagerringe 31, 32, 33 sind koaxial zueinander ausgerichtet.

Im Betrieb wird in den Führungskörper 22 ein rotierendes Bearbeitungswerkzeug 37 eingeschoben, beispielsweise ein Fingerfräser, dessen Außendurchmesser dem Innendurchmesser der Lagerringe 34, 35, 36 entspricht, so daß das Bearbeitungswerkzeug 37 in den inneren Lagerringen 34, 35, 36 koaxial zum Führungskörper 22 geführt ist. Bei der Drehung des Bearbeitungswerkzeuges 37 drehen sich die inneren Lagerringe 34, 35, 36 mit, so daß die Relativverschiebung zwischen dem Bearbeitungswerkzeug 37 und den das Bearbeitungswerkzeug 37 stützenden inneren Lagerringen 34, 35 und 36 gering ist.

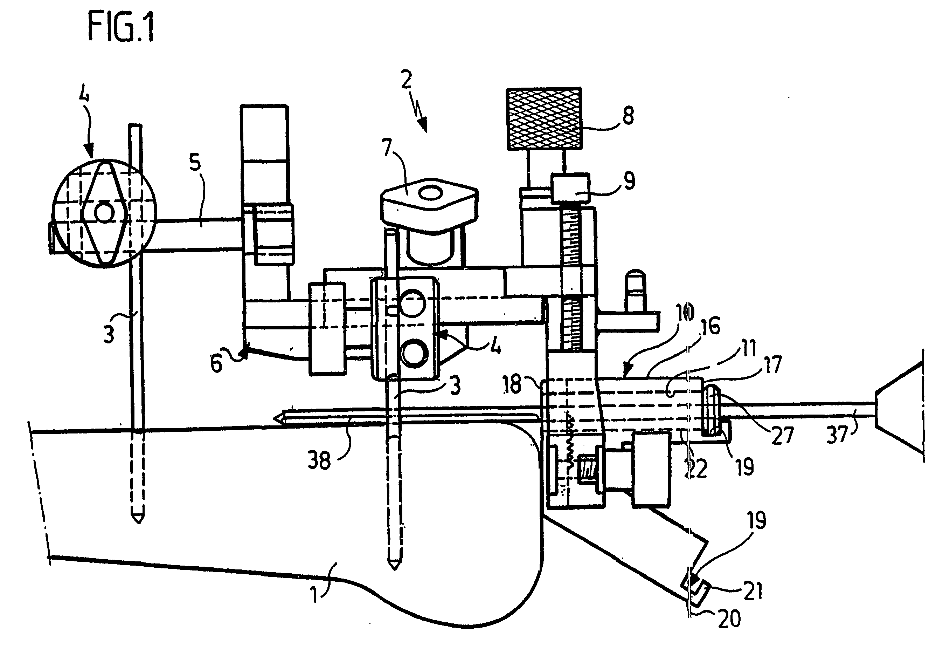

Das Bearbeitungswerkzeug 37 ragt an der Rückseite 18 aus dem Führungskörper 22 heraus und kann dort mit seinen Schneidflächen 38 den Knochen 1 bearbeiten (Figur 1). Dabei kann der Operateur das Bearbeitungswerkzeug 37 in der durch den Führungsschlitz 11 definierten Ebene quer zur Längsrichtung des Bearbeitungswerkzeuges 37 verschieben und um eine senkrecht dazu verlaufende Achse verschwenken, so daß die gesamte zu bearbeitende Fläche des Knochens 1 von dem Bearbeitungswerkzeug 37 überstrichen und bearbeitet werden kann. Der Führungskörper 22 folgt der jeweiligen Bewegung des Bearbeitungswerkzeuges 37 in dieser Ebene und führt das Bearbeitungswerkzeug 37 gegen eine Verschwenkung aus der vom Führungsschlitz 11 vorgegebenen Ebene heraus. Durch die Lagerung des Bearbeitungswerkzeuges 37 in den inneren Lagerringen 34, 35, 36 wird auch bei hoher Drehzahl keine Abnutzung des Bearbeitungswerkzeuges 37 oder des Führungsschlitzes 11 auftreten, dasselbe gilt hinsichtlich unerwünscht hoher Erwärmungen.

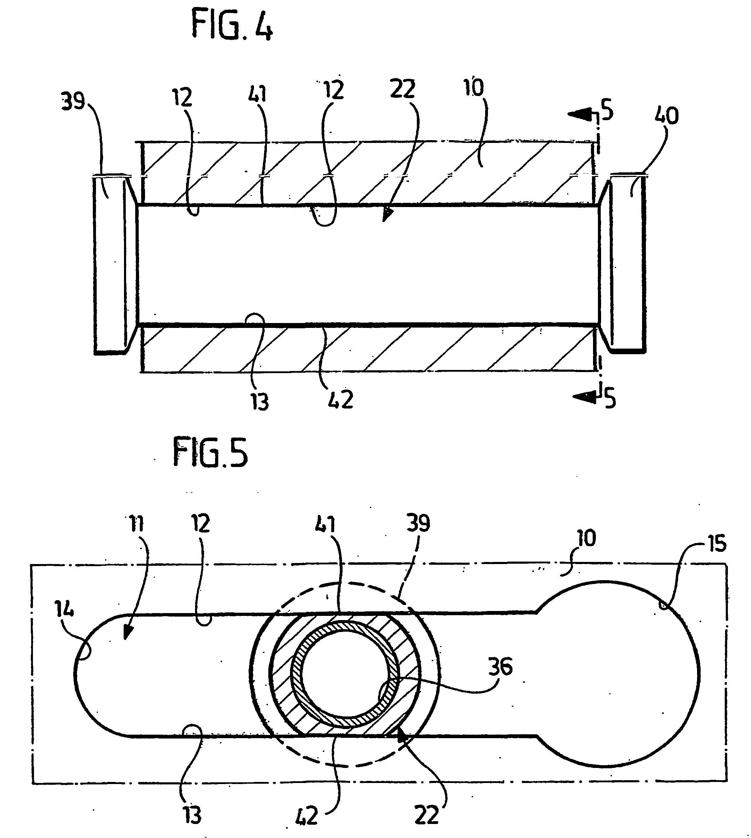

Während bei dem in den Figuren 1 bis 3 dargestellten Ausführungsbeispiel der Führungskörper 22 in axialer Richtung durch die in die Nut 19 eintauchende Verbreiterung 27 gesichert wird, erfolgt diese axiale Sicherung bei dem Ausführungsbeispiel der Figuren 4 und 5, das ansonsten ähnlich aufgebaut ist und bei dem gleiche Teile dieselben Bezugszeichen tragen, durch ringflanschförmige Verbreiterungen 39 und 40 an beiden Enden des Führungskörpers 22.

Der Führungskörper 22 steht bei diesem Ausführungsbeispiel auf beiden Seiten aus dem Führungsschlitz 11 hervor, und die ringflanschförmigen Verbreiterungen 39 und 40, die vorzugsweise abgeschrägt sind, sichern den Führungskörper 22 in axialer Richtung mit so viel Spiel, daß auch eine Verschwenkung des Führungskörpers 22 in der Ebene des Führungsschlitzes 11 möglich ist. Bei diesem Ausführungsbeispiel kann daher auf die Nut 19 verzichtet werden.

Bei diesem Ausführungsbeispiel ist der Führungskörper 22 an seiner Oberseite und an seiner Unterseite abgeflacht, diese Abflachungen 41 und 42 liegen dabei an den Seitenflächen 12 und 13 des Führungsschlitzes 11 an und verhindern eine Verdrehung des Führungskörpers 22 um seine Längsachse.

An einem Ende weisen die Seitenflächen 12 und 13 des Führungsschlitzes 11 einen größeren Abstand voneinander auf, der Abstand ist dabei so groß, daß die Verbreiterung 39, die Verbreiterung 40 oder auch beide Verbreiterungen durch sie hindurchpassen, es ist damit möglich, in diesem Endbereich den Führungskörper 22 in den Führungsschlitz 11 einzuschieben oder wieder aus diesem herauszuziehen.

## Patentansprüche

1. Führung für ein mittels einer Bearbeitungsschablone (2) in einer Schlitzführung (11) geführtes, drehbares chirurgisches Bearbeitungswerkzeug (37), wobei sie einen Führungskörper (22) umfaßt, dessen Höhe der der Schlitzführung (11) entspricht, während seine Breite geringer ist als die Breite der Schlitzführung (11), daß der Führungskörper (22) eine durchgehende Aufnahmebohrung (30) für das Bearbeitungswerkzeug (37) aufweist, **dadurch gekennzeichnet, daß** in der Aufnahmebohrung (30) mindestens eine an dem Bearbeitungswerkzeug (37) anliegende Führungsfläche (34, 35, 36) angeordnet ist, die koaxial zur Drehachse des Bearbeitungswerkzeuges (37) drehbar in dem Führungskörper (22) gelagert ist.

2. Führung nach Anspruch 1, **dadurch gekennzeichnet, daß** in der Aufnahmebohrung (30) zwei drehbare Führungsflächen (34, 35, 36) im Abstand zueinander angeordnet sind.

3. Führung nach Anspruch 1, **dadurch gekennzeichnet, daß** in der Aufnahmebohrung (30) drei drehbare Führungsflächen (34, 35, 36) im Abstand zueinander angeordnet sind.

4. Führung nach einem der voranstehenden Ansprüche, **dadurch gekennzeichnet, daß** die Führungsfläche durch die Innenwand eines inneren Lagerringes (34, 35, 36) eines Wälzlagers (31, 32, 33) gebildet wird.

5. Führung nach Anspruch 4, **dadurch gekennzeichnet, daß** das Wälzlager ein Kugellager (31, 32, 33) ist.

6. Führung nach einem der voranstehenden Ansprüche, **dadurch gekennzeichnet, daß** der Führungskörper (22) an seiner Oberseite und/oder an seiner Unterseite Reibflächen (25, 26) aufweist, die reibend an den einander gegenüberliegenden Seitenflächen (12, 13) der Schlitzführung (11) der Bearbeitungs schablone (10) anliegen.

7. Führung nach Anspruch 6, **dadurch gekennzeichnet, daß** die Reibflächen durch elastisch zusammendrückbare Teilbereiche (25, 26) der Oberseite und/oder der Unterseite des Führungskörpers (22) gebildet werden.

8. Führung nach Anspruch 7, **dadurch gekennzeichnet, daß** die Reibflächen durch gummielastische O-Ringe (25, 26) gebildet werden, die in quer zur Längsrichtung des Führungskörpers (22) umlaufenden Umfangsnuten (23, 24) des Führungskörpers (22) eingelegt sind.

9. Führung nach einem der voranstehenden Ansprüche, **dadurch gekennzeichnet, daß** der Führungskörper (22) durch mindestens einen in einen Rücksprung (19) eingreifenden Vorsprung (27) relativ zum Führungsschlitz (11) gegen eine Verschiebung in axialer Richtung gesichert ist.

10. Führung nach Anspruch 9, **dadurch gekennzeichnet, daß** der Rücksprung (19) und der Vorsprung (27) derart ineinander eingreifen, daß der Führungskörper (22) relativ zum Führungsschlitz (11) quer zu dessen Längsrichtung verschiebbar und um eine senkrecht auf dem Führungsschlitz (11) stehende Drehachse verschwenkbar ist.

11. Führung nach einem der Ansprüche 9 oder 10, **dadurch gekennzeichnet, daß** der Vorsprung eine flanschförmige Verbreiterung (27) am Führungskörper (22) ist, die in eine parallel zum Führungsschlitz (11) verlaufende Nut (19) an der Bearbeitungsschablone (10) eintaucht.

12. Führung nach Anspruch 11, **dadurch gekennzeichnet, daß** die Verbreiterung (27) derart abgeschrägt ist, daß der Führungskörper (22) in der Ebene des Führungsschlitzes (11) relativ zu der Nut (19) verschwenkbar ist.

13. Führung nach einem der Ansprüche 11 oder 12, **dadurch gekennzeichnet, daß** die Seitenwand (21) der Nut (19) an einer Stelle fehlt, so daß die Verbreiterung (27) an dieser Stelle seitlich aus der Nut (19) austreten kann.

14. Führung nach einem der Ansprüche 1 bis 8, **dadurch gekennzeichnet, daß** der Führungskörper (22) an seinen beiden Enden aus dem Führungsschlitz (11) hervorragt und dort Verbreiterungen (39, 40) trägt, die den Führungskörper (22) gegen eine axiale Verschiebung im Führungsschlitz (11) sichern.

15. Führung nach Anspruch 14, **dadurch gekennzeichnet, daß** die Verbreiterungen (39, 40) mit Spiel an der Vorder- und Rückseite (17, 18) des Führungsschlitzes (11) angeordnet sind, so daß der Führungskörper (22) in der Ebene des Führungsschlitzes (11) um eine senkrecht auf dieser Ebene stehende Drehachse verschwenkbar ist.

16. Führung nach einem der Ansprüche 14 oder 15, **dadurch gekennzeichnet, daß** der Führungsschlitz (11) an einer Stelle einen vergrößerten Abstand der einander gegenüberliegenden Seitenflächen (12, 13) aufweist, so daß an dieser Stelle zumindest eine der Verbreiterungen (39, 40) durch den Führungsschlitz (11) hindurchtreten kann.

17. Führung nach einem der voranstehenden Ansprüche, **dadurch gekennzeichnet, daß** der Führungskörper (22) als kreiszylindrische Führungshülse ausgebildet ist.

18. Führung nach Anspruch 17, **dadurch gekennzeichnet, daß** der Führungskörper (22) einen von der kreisförmigen Querschnittsform abweichenden Sicherungsabschnitt (28; 41, 42) aufweist, der an einer Anlagefläche (29; 12, 13) der Bearbeitungsschablone (10 anliegt und eine Verdrehung des Führungskörpers (22) um seine Längsachse verhindert.

19. Führung nach einem der Ansprüche 11 oder 12 und nach Anspruch 18, **dadurch gekennzeichnet, daß** der Sicherungsabschnitt (28) an der flanschförmigen Verbreiterung (27) angeordnet ist und an der Nut (29, 19) anliegt.

20. Führung nach Anspruch 18, **dadurch gekennzeichnet, daß** der Führungskörper (22) an der Oberseite und/oder der Unterseite abgeflacht ausgebildet ist und daß diese an den gegenüberliegenden Seitenflächen (12, 13) des Führungsschlitzes (11) anliegenden Abflachungen (41, 42) den Sicherungsabschnitt bilden.

## Claims

1. A guide for a rotatable surgical machining tool (37) guided in a slotted guide (11) by means of a machining gauge (2), wherein the guide comprises a guide body (22), the height of which corresponds to that of the slotted guide (11), while its width is smaller than the width of the slotted guide (11), and wherein the guide body (22) has a continuous receiving bore (30) for the machining tool (37), **characterised in that** at least one guide surface (34, 35, 36), which rests against the machining tool (37), is arranged in the receiving bore (30) and is mounted in the guide body (22) so as to be rotatable co-axially with the rotation axis of the machining tool (37).

2. A guide according to claim 1, **characterised in that** two rotatable guide surfaces (34, 35, 36) are arranged spaced apart in the receiving bore (30).

3. A guide according to claim 1, **characterised in that** three rotatable guide surfaces (34, 35, 36) are arranged spaced apart in the receiving bore (30).

4. A guide according to any one of the preceding claims, **characterised in that** the guide surface is formed by the inner wall of an inner bearing race (34, 35, 36) of a rolling bearing (31, 32, 33).

5. A guide according to claim 4, **characterised in that** the rolling bearing is a ball bearing (31, 32, 33).

6. A guide according to any one of the preceding claims, **characterised in that** the guide body (22) has, on its upper side and/or on its underside, friction surfaces (25, 26) which rest frictionally against the opposing lateral surfaces (12, 13) of the slotted guide (11) of the machining gauge (10).

7. A guide according to claim 6, **characterised in that** the friction surfaces are formed by resiliently compressible sub-regions (25, 26) of the upper side and/or the underside of the guide body (22).

8. A guide according to claim 7, **characterised in that** the friction surfaces are formed by resilient O-rings (25, 26) placed into circumferential grooves (23, 24) of the guide body (22), said circumferential grooves (23, 24) extending transversely to the longitudinal direction of the guide body (22).

9. A guide according to any one of the preceding claims, **characterised in that** the guide body (22) is secured against axial displacement relative to the guide slot (11) by means of at least one projection (27) engaging in a recess (19).

10. A guide according to claim 9, **characterised in that** the recess (19) and the projection (27) mutually engage in such a way that the guide body (22) is displaceable relative to the guide slot (11) transversely to the longitudinal direction thereof and is pivotable about a rotation axis extending perpendicularly to the guide slot (11).

11. A guide according to either one of claims 9 and 10, **characterised in that** the projection is a flange-type widening (27) provided on the guide body (22) and extending into a groove (19) in the machining gauge (10), the groove (19) extending parallel to the guide slot (11).

12. A guide according to claim 11, **characterised in that** the widening (27) is chamfered so that the guide body (22) is pivotable relative to the groove (19) in the plane of the guide slot (11).

13. A guide according to either one of claims 11 and 12, **characterised in that** the side wall (21) of the groove (19) is missing at one point so that the widening (27) can project laterally from the groove (19) at this point.

14. A guide according to any one of claims 1 to 8, **characterised in that** both ends of the guide body (22) project from the guide slot (11) and carry widenings (39, 40) which secure the guide body (22) against axial displacement in the guide slot (11).

15. A guide according to claim 14, **characterised in that** the widenings (39, 40) are arranged with clearance at the front and rear end (17, 18) of the guide slot (11) so that the guide body (22) is pivotable in the plane of the guide slot (11) about a rotation axis extending perpendicularly to this plane.

16. A guide according to either one of claims 14 and 15, **characterised in that** the guide slot (11) has a greater distance between its opposing lateral surfaces (12, 13) at one point so that at least one of the widenings (39, 40) can pass through the guide slot (11) at this point.

17. A guide according to any one of the preceding claims, **characterised in that** the guide body (22) is formed as a circular cylindrical guide sleeve.

18. A guide according to claim 17, **characterised in that** the guide body (22) has a securing portion (28; 41, 42) which deviates from the circular cross-sectional shape and which rests against a contact surface (29; 12, 13) of the machining gauge (10) and prevents rotation of the guide body (22) about its longitudinal axis.

19. A guide according to either one of claims 11 and 12 and according to claim 18, **characterised in that** the securing portion (28) is arranged on the flange-type widening (27) and rests against the groove (29, 19).

20. A guide according to claim 18, **characterised in that** the guide body (22) is flattened on its upper side and/or underside and **in that** these flattened regions (41, 42), which rest against the opposing lateral surfaces (12, 13) of the guide slot (11), form the securing portion.

## Revendications

1. Guidage pour un outil d'usinage chirurgical rotatif (37), guidé dans un guidage à rainure (11) au moyen d'un gabarit d'usinage (2), qui comprend un corps de guidage (22) dont la hauteur correspond à celle du guidage à rainure (11), tandis que sa largeur est inférieure à la largeur du guidage à rainure (11), en ce que le corps de guidage (22) comporte un perçage de positionnement traversant (30) pour l'outil d'usinage (37), **caractérisé en ce que** dans le perçage de positionnement (30) est disposée au moins une surface de guidage (34, 35, 36) voisine de l'outil d'usinage (37), qui est montée à rotation dans le corps de guidage (22) coaxialement à l'axe de rotation de l'outil d'usinage (37).

2. Guidage selon la revendication 1, **caractérisé en ce que** deux surfaces de guidage rotatives (34, 35, 36) sont disposées à distance l'une de l'autre dans le perçage de positionnement (30).

3. Guidage selon la revendication 1, **caractérisé en ce que** trois surfaces de guidage rotatives (34, 35, 36) sont disposées à distance les unes des autres dans le perçage de positionnement (30).

4. Guidage selon l'une des revendications précédentes, **caractérisé en ce que** la surface de guidage est formée par la paroi interne d'une bague de roulement interne (34, 35, 36) d'un roulement (31, 32, 33).

5. Guidage selon la revendication 4, **caractérisé en ce que** le roulement est un roulement à billes (31, 32, 33).

6. Guidage selon l'une des revendications précédentes, **caractérisé en ce que** le corps de guidage (22) comporte au niveau de son côté supérieur et/ou au niveau de son côté inférieur des surfaces de frottement (25, 26) qui reposent à frottement contre les surfaces latérales (12, 13) opposées l'une à l'autre du guidage à rainure (11) du gabarit d'usinage (10).

7. Guidage selon la revendication 6, **caractérisé en ce que** les surfaces de frottement sont formées par des domaines partiels élastiquement compressibles (25, 26) du côté supérieur et/ou du côté inférieur du corps de guidage (22).

8. Guidage selon la revendication 7, **caractérisé en ce que** les surfaces de frottement sont formées par des joints toriques élastiques (25, 26) qui sont placés dans des rainures périphériques (23, 24) du corps de guidage (22) qui s'étendent transversalement à la direction longitudinale du corps de guidage (22).

9. Guidage selon l'une des revendications précédentes, **caractérisé en ce que** le corps de guidage (22) est bloqué contre un déplacement en direction axiale par rapport à la rainure de guidage (11) par au moins une saillie (27) qui pénètre dans un retrait (19).

10. Guidage selon la revendication 9, **caractérisé en ce que** le retrait (19) et la saillie (27) pénètrent l'un dans l'autre de telle manière que le corps de guidage (22) est déplaçable par rapport à la rainure de guidage (11) transversalement à sa direction longitudinale et peut pivoter autour d'un axe de rotation situé perpendiculairement sur la rainure de guidage (11).

11. Guidage selon l'une des revendications 9 ou 10, **caractérisé en ce que** la saillie est un élargissement en forme de bride 27 sur le corps de guidage (22) qui pénètre dans une rainure (19) qui s'étend parallèlement à la rainure de guidage (11) sur le gabarit d'usinage (10).

12. Guidage selon la revendication 11, **caractérisé en ce que** l'élargissement (27) est chanfreiné de telle manière que le corps de guidage (22) peut pivoter par rapport à la rainure (19) dans le plan de la rainure de guidage (11).

13. Guidage selon l'une des revendications 11 ou 12, **caractérisé en ce que** la paroi latérale (21) de la rainure (19) est manquante à un endroit de sorte que l'élargissement (27) peut sortir latéralement de la rainure (19) à cet endroit.

14. Guidage selon l'une des revendications 1 à 8, **caractérisé en ce que** le corps de guidage (22) fait saillie de la rainure de guidage (11) au niveau de ses deux extrémités et y porte des élargissements (39, 40) qui bloquent le corps de guidage (22) contre un déplacement axial dans la rainure de guidage (11).

15. Guidage selon la revendication 14, **caractérisé en ce que** les élargissements (39, 40) sont disposés avec un jeu au niveau du côté antérieur et du côté postérieur (17, 18) de la rainure de guidage (11), de sorte que le corps de guidage (22) peut pivoter dans le plan de la rainure de guidage (11) autour d'un axe de rotation situé perpendiculairement sur ce plan.

16. Guidage selon l'une des revendications 14 ou 15, **caractérisé en ce que** la rainure de guidage (11) présente à un endroit une plus grande distance des surfaces latérales opposées l'une à l'autre (12, 13) de sorte qu'à cet endroit au moins l'un des élargissements (39, 40) peut traverser la rainure de guidage (11).

17. Guidage selon l'une des revendications précédentes, **caractérisé en ce que** le corps de guidage (22) est sous forme de douille de guidage cylindrique circulaire.

18. Guidage selon la revendication 17, **caractérisé en ce que** le corps de guidage (22) comporte un segment d'arrêt (28 ; 41, 42) qui s'écarte de la forme en section transversale circulaire, qui est voisin d'une surface d'appui (29 ; 12, 13) du gabarit d'usinage (10) et qui empêche une rotation du corps de guidage (22) autour de son axe longitudinal.

19. Guidage selon l'une des revendications 11 ou 12 et selon la revendication 18, **caractérisé en ce que** le segment d'arrêt (28) est disposé au niveau de l'élargissement en forme de bride (27) et est voisin de la rainure (29, 19).

20. Guidage selon la revendication 18, **caractérisé en ce que** le corps de guidage (22) est aplati au niveau du côté supérieur et/ou du côté inférieur et **en ce que** ces aplatissements (41, 42) voisins des surfaces latérales opposées l'une à l'autre (12, 13) de la rainure de guidage (11) forment le segment d'arrêt.
